(19) Europäisches Patentamt
European Patent Office
Office européen des brevets

(11) Veröffentlichungsnummer: **0 118 854**
**B1**

(12) # EUROPÄISCHE PATENTSCHRIFT

(45) Veröffentlichungstag der Patentschrift: **23.07.86**

(21) Anmeldenummer: **84102281.7**

(22) Anmeldetag: **02.03.84**

(51) Int. Cl.⁴: **A 01 N 43/60,** A 01 N 57/24, C 07 D 215/56, C 07 D 401/04, C 07 D 405/12, C 07 D 405/14, C 07 F 9/60

(54) **Chinoloncarbonsäure enthaltende bakterizide Mittel.**

(30) Priorität: **12.03.83 DE 3308908**

(43) Veröffentlichungstag der Anmeldung: **19.09.84 Patentblatt 84/38**

(45) Bekanntmachung des Hinweises auf die Patenterteilung: **23.07.86 Patentblatt 86/30**

(84) Benannte Vertragsstaaten: **AT BE CH DE FR GB IT LI NL**

(56) Entgegenhaltungen:
**EP-A-0 049 355**
**FR-A-2 449 682**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Kuck, Karl- Heinz, Dr., Heerstrasse 24, D-4018 Langenfeld (DE)**

**EP 0 118 854 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft die Verwendung von Chinoloncarbonsäuren als antibakterielle Mittel.

Es sind bereits bestimmte 1-Cyclopropyl-1,4-dihydro-4-oxo-3-chinolincarbonsäuren, wie z.B. die 1-Cyclopropyl-6-fluor-1,4-dihydro-4-oxo-7-(1-piperazinyl)-3-chinolincarbonsäure, bekannt (vgl. Europäische Patentschrift 49 355). Über eine mikrobizide Wirkung auf dem Pflanzenschutzgebiet ist nichts bekannt, nur ihre Verwendung auf dem Pharmagebiet.

Weiterhin ist behkannt geworden, daß bestimmte Chinoloncarbonsäurederivate, wie z.B. die 7-Chlor-1-ethyl-6-fluor-1,4-dihydro-4-oxo-3-chinolincarbonsäure, eine bakterizide Wirkung auf dem Pflanzenschutzgebiet aufweisen (vgl. Europäische Patentanmeldung 0203). Die Wirksamkeit ist jedoch bei niedrigen Anwendungskonzentrationen nicht immer befriedigend.

Es wurde die neue Verwendung der Chinoloncarbonsäuren der Formel (I)

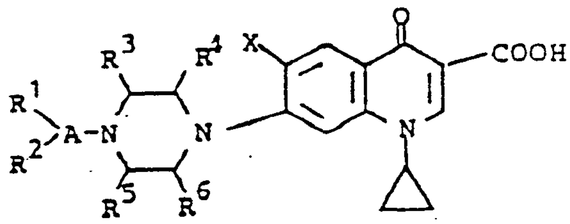

(I)

in der

A für geradkettiges oder verzweites Alkylen mit 1 bis 6 Kohlenstoffatomen oder einen Rest >C=CH—,

$R^1$ für Alkoxycarbonyl mit 1 bis 6 Kohlenstoffatomen im Alkylteil, Benzyloxycarbonyl, Carboxy, gegebenenfalls substituiertes Carbamoyl, Cyano, Dialkoxyphosphonyl oder Alkylsulfonyl mit 1 bis 4 Kohlenstoffatomen im Alkylteil und

$R^2$ für Wasserstoff, Alkoxycarbonyl mit 1 bis 6 Kohlenstoffatomen im Alkylteil, Benzyloxycarbonyl, gegebenenfalls substituiertes Carbamoyl, Cyano, Chlor, Acetyl, Alkyl mit 1 bis 3 Kohlenstoffatomen oder Phenyl stehen, wobei $R^1$ und $R^2$ auch gemeinsam mit dem Kohlenstoffatom, an das sie gebunden sind einen 2-Oxo-tetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen, vorzugsweise Fluor oder Chlor, oder Nitro steht,

und deren Säureadditions-, Alkali- und Erdalkalisalze, Zink-, Mangan- und Kupfersalze und Hydrate zur Bekämpfung von Bakterien im Pflanzenschutz gefunden.

Gegenstand der Erfindung sind auch die Zink-, Mangan- und Kupfersalze der Formel (I) als solche, wie sie in den Stoffansprüchen definert sind.

Überraschenderweise zeigen die erfindungsgemäß verwendbaren Chinoloncarbonsäuren der Formel (I) eine höhere bakterizide Wirksamkeit als die aus dem Stand der Technik chemisch und wirkungsmäßig nächstliegende Chinoloncarbonsäure, die 7-Chlor-1-ethyl-6-fluor-1,4-dihydro-4-oxo-3-chinoloncabonsäure. Die erfindungsgemäße neue Verwendung der Verbindungen stellt somit eine Bereicherung der Technik dar.

Die erfindungsgemäß verwendbaren Vewrbindungen sind durch die Formel (I) allgemein defineirt.

Bevorzugt ist die Verwendung solcher Verbindungen der Formel (I), in denen

A für geradkettiges oder verzweigtes Akylen mit 1 bis 5 Kohlenstoffatomen oder die Gruppe >C=CH— steht,

$R^1$ für Alkoxycarbonyl mit 1 bis 5 Kohlenstoffatomen im Alkylteil, Benzylcarbonyl, Carboxy, gegebenefalls durch 1.oder 2 Methyl- oder Ethylreste substituiertes Carbamoyl, Cyano, Methyl- oder Ethylsulfonyl steht,

$R^2$ für Wasserstoff, Alkoxycarbonyl mit 1 bis 5 Kohlenstoffatomen im Alkylteil, Benzyloxycarbonyl, Carbamoyl, Cyano, Chlor, Acetyl, Alkyl mit 1 oder 2 Kohlenstoffatomen oder Phenyl steht, wobei $R^1$ und $R^2$ auch gemeinsam mit dem Kohlenstoffatom, das sie substituieren, einen 2-Oxo-tetrahydrofurylring bilden können,

$R^3$, $R^4$, $R^5$ und $R^6$ für Wasserstoff, Methyl oder Ethyl stehen und

X für Wasserstoff, Fluor, Chlor oder Nitro steht.

Besonders bevorzugt ist die Verwendung solcher Verbindungen der Formel (I), in denen

A für geradkettiges Alkylen mit 1 bis 5 Kohlenstoffatomen oder die Gruppe >C=CH— steht,

$R^1$ für Alkoxycarbonyl mit 1 bis 4 Kohlenstoffatomen im Alkylteil, Benzyloxycarbonyl, Carboxy, Carbamoyl, Cyano oder Methylsulfonyl steht,

$R^2$ für Wasserstoff, Alkoxycarbonyl mit 1 bis 3 Kohlenstoffatomen im Alkylteil, Cyano, Chlor, Acetyl oder Phenyl steht, wobei $R^1$ und $R^2$ auch gemeinsam mit dem Kohlenstoffatom, das sie substituieren, einen 2-Oxo-tetrahydro-3-furylring bilden können,

$R^3$ für Wasserstoff, Methyl oder Ethyl steht,

$R^4$ für Wasserstoff steht,

$R^5$ für Wasserstoff oder Methyl steht,
$R^6$ für Wasserstoff und
X für Wasserstoff, Fluor, Ch lor oder Nitro stehen.

Die erfindungsgemäß verwendbaren Verbindungen der Formel (I) können gegebenenfalls mit einer organischen oder anorganischen Säure in ein Salz übergeführt werden. Zur Salzbildung geeignete Säuren sind z.B. Halogenwasserstoffsäuren, wie Salzsäure, Bromwasserstoffsäure, Iodwasserstoffsäure, ferner Schwefelsäure, Essigsäure, Zitronensäure, Ascorbinsäure, Methansulfonsäure und Benzolsulfonsäure. Als Alkali- bzw. Erdalkalisalze sind vorzungsweise Natrium-, Kalium-, Calcium- und Magnesiumsalze geeignet, außerdem sind Kupfer-, Zink- und Mangansalze geeignet.

Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Als im Pflanzenschutz antibakteriell verwendbare Wirkstoffe seien im einzelnen genannt:

7-[4-(Methoxycarbonylmethyl)-1-piperazinyl]-1-cyclopropyl-6-fluor-1,4-dihydro-4-oxo-3-chinolincarbonsäure,

7-[4-(Ethoxycarbonylmethyl)-1-piperazinyl]-1-cyclopropyl-6-fluor-1,4-dihydro-4-oxo-3-chinolincarbonsäure,

7-[4-(2-Benzyloxycarbonylethyl)-1-piperazinyl]-1-cyclopropyl-6-fluor-1,4-dihydro-4-oxo-3-chinolincarbonsäure,

7-[4-(5-Benzyloxycarbonylpentyl)-1-piperazinyl]-1-cyclopropyl-6-fluor-1,4-dihydro-4-oxo-3-chinolincarbonsäure,

7-[4-(2-Methoxycarbonylethyl)-1-piperazinyl]-1-cyclopropyl-6-fluor-1,4-dihydro-4-oxo-3-chinolincarbonsäure,

7-[4-(2-Ethoxycarbonylethyl)-1-piperazinyl]-1-cyclopropyl-6-fluor-1,4-dihydro-4-oxo-3-chinolincarbonsäure,

7-[4-(2-Propyloxycarbonylethyl)-1-piperazinyl]-1-cyclopropyl-6-fluor-1,4-dihydro-4-oxo-3-chinolincarbonsäure,

7-[4-(2-n-Butoxycarbonylethyl)-1-piperazinyl]-1-cyclopropyl-6-fluor-1,4-dihydro-4-oxo-3-chinolincarbonsäure,

7-[4-(2-Cyanoethyl)-1-piperazinyl]-1-cyclopropyl-6-fluor-1,4-dihydro-4-oxo-3-chinolincarbonsäure,

7-[4-(3-Cyanopropyl)-1-piperazinyl]-1-cyclopropyl-6-fluor-1,4-dihydro-4-oxo-3-chinolincarbonsäure,

7-{4-[α-(Benzyloxycarbonyl)-benzyl]-1-piperazinyl}-1-cyclopropyl-6-fluor-1,4-dihydro-4-oxo-3-chinolincarbonsäure,

7-[4-Carbamoylmethyl-1-piperazinyl]-1-cyclopropyl-6-fluor-1,4-dihydro-4-oxo-3-chinolincarbonsäure,

7-[4-Cyanomethyl-1-piperazinyl]-1-cyclopropyl-6-fluor-1,4-dihydro-4-oxo-3-chinolincarbonsäure,

7-[4-(N-Methylcarbamoylmethyl)-1-piperazinyl]-1-cyclopropyl-6-fluor-1,4-dihydro-4-oxo-3-chinolincarbonsäure,

7-[4-(N-Ethylcarbamoylmethyl)-1-piperazinyl]-1-cyclopropyl-6-fluor-1,4-dihydro-4-oxo-3-chinolincarbonsäure,

7-{4-[2-Oxo-1-(methoxycarbonyl)-1-propyl]-1-piperazinyl}-1-cyclopropyl-6-fluor-1,4-dihydro-4-oxo-3-chinolincarbonsäure,

7-[4-(2-Oxo-tetrahydrofuryl-3-)-1-piperazinyl]-1-cyclopropyl-6-fluor-1,4-dihydro-4-oxo-3-chinolincarbonsäure,

7-[4-(Carboxymethyl)-1-piperazinyl]-1-cyclopropyl-6-fluor-1,4-dihydro-4-oxo-3-chinolincarbonsäure,

7-[4-(2-Carboxyethyl)-1-piperazinyl]-1-cyclopropyl-6-fluor-1,4-dihydro-4-oxo-3-chinolincarbonsäure,

7-[4-(2-Carboxypropyl)-1-piperazinyl]-1-cyclopropyl-6-fluor-1,4-dihydro-4-oxo-3-chinolincarbonsäure,

7-[4-(3-Carboxypropyl)-1-piperazinyl]-1-cyclopropyl-6-fluor-1,4-dihydro-4-oxo-3-chinolincarbonsäure,

7-[4-(5-Carboxypentyl)-1-piperazinyl]-1-cyclopropyl-6-fluor-1,4-dihydro-4-oxo-3-chinolincarbonsäure,

7-[4-(α-Carboxybenzyl)-1-piperazinyl]-1-cyclopropyl-6-fluor-1,4-dihydro-4-oxo-3-chinolincarbonsäure,

7-[4-(2-Chlor-2-cyanoethyl)-1-piperazinyl]-1-cyclopropyl-6-fluor-1,4-dihydro-4-oxo-3-chinolincarbonsäure,

7-[4-(2-Methylsulfonyl-ethyl)-1-piperazinyl]-1-cyclopropyl-6-fluor-1,4-dihydro-4-oxo-3-chinolincarbonsäure,

7-[4-(2-Diethoxyphosphonyl-ethyl)-1-piperazinyl]-1-cyclopropyl-6-fluor-1,4-dihydro-4-oxo-3-chinolincarbonsäure,

7-[4-(2-Methoxycarbonylethyl)-1-piperazinyl]-1-cyclopropyl-1,4-dihydro-6-nitro-4-oxo-3-chinolincarbonsäure,

7-[4-(2-Methoxycabonylethyl)-1-piperazinyl]-6-chlor-1-cyclopropyl-1,4-dihydro-4-oxo-3-chinolincarbonsäure,

7-[4-(2-Methoxycarbonylethyl)-1-piperazinyl]-1-cyclopropyl-1,4-dihydro-4-oxo-3-chinolincarbonsäure,

7-[4-(2-Benzyloxycarbonylethyl)-3-methyl-1-piperazinyl]-1-cyclopropyl-6-fluor-1,4-dihydro-4-oxo-3-chinolincarbonsäure,

7-[4-(2-Methoxycarbonylethyl)-3,5-dimethyl-1-piperazinyl]-1-cyclopropyl-6-fluor-1,4-dihydro-4-oxo-3-chinolincarbonsäure,

Auch diese erfindungsgemäß verwendbaren Verbindungen der Formel (I) können wie besprochen gegebenenfalls mit einer organischen oder anorganischen Säure in ein Salz übergeführt werden. Zur Salzbildung geeignete Säuren sind die schon erwähnten Halogenwasserstoffsäuren, wie Salzsäure, Bromwasserstoffsäure, Iodwasserstoffsäure, ferner Schwefelsäure, Essigsäure, Zitronensäure, Ascorbinsäure und Benzolsulfonsäure. Als Alkali- bzw. Erdalkalisalze sind vorzungsweise Natrium-, Kalium-, Calcium- und Magnesiumn-salze geeignet und die Kupfer-, Zink- und Mangansalze.

Die erfindungsgemäß zu verwendenden Wirkstoffe sind Gegenstand einer eigenen älteren Patentanmeldung, die veröffentlicht ist als EP-Anmeldung 84 101 443.4 (Veröffenthiclungsnummer 0117474). Sie werden erhalten, wenn man eine Verbindung der Formel (II)

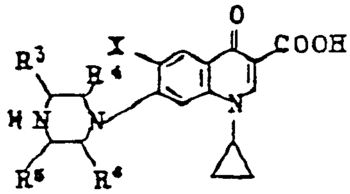

(II)

in welcher

X, $R^3$, $R^4$ und $R^5$ und $R^6$ die oben angegebene Bedeutung haben,

mit einer Verbindung der Formel (III)

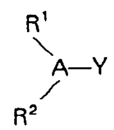

III

in welcher

$R^1$, $R^2$ und A die oben angegebene Bedeutung haben und

Y für Halogen, vorzungsweise Chlor, Brom oder Iod, $CH_3O—SO_2—O—$, $C_2H_5O—SO_2—O—$, Methoxy oder Ethoxy steht, umsetzt (Methode A).

Man erhält erfindungsgemäß verwendbare Verdindungen der Formel (I) auch, wenn man Verbindungen der Formel (II)

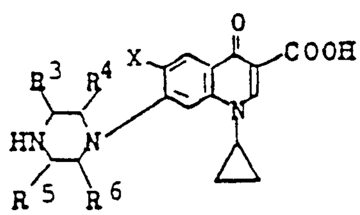

(II)

mit Verbindungen der Formel (IV)

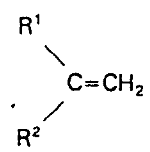

IV

in welcher

$R^1$ und $R^2$ die obern angegebene Bedeutung haben,

umsetzt, wobei erfindungsgemäß verwendbare Verbindungen der Formel (Ia)

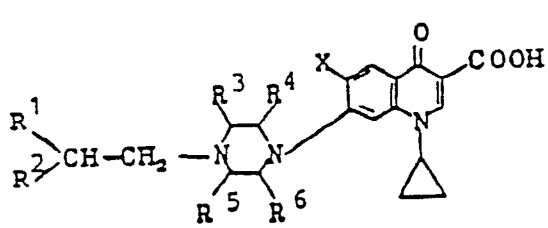

(Ia)

entstehen (Methode B).

Man erhält erfindungsgemäß verwendbare Verbindung der Formel (I) auch, wenn man Verbindung der Formel (V)

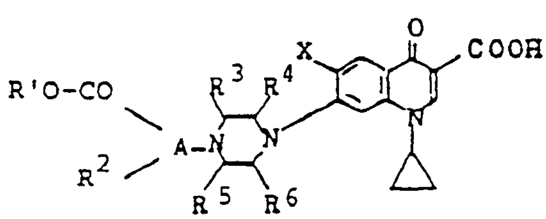

(V)

in welcher

$R^2$, $R^3$, $R^4$, $R^5$, $R^6$ und X die oben angegebene Bedeutung haben und

R' für Alkyl mit 1 bis 6 Kohlenstoffatomen oder Benzyl steht,

unter alkalischen oder sauren Bedingungen oder im Falle von R' für Benzyl auch unter hydrogenolytischen Bedingungen umsetzt, wobei erfindungsgemäß verwendbare Verbindungen der Formel (Ib) entstehen (Methode C)

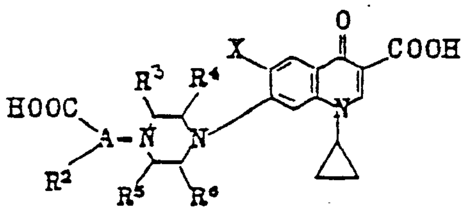

(Ib)

in welcher

$R^2$, $R^3$, $R^4$, $R^5$, $R^6$ und X die oben angegebene Bedeutung haben.

Die als Ausgangsverbindungen einzusetzenden Verbindungen der Formel (II) können durch Umsetzung von Verbindungen der Formel (VI)

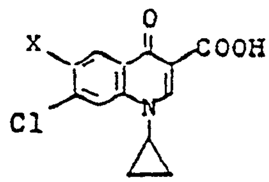

(VI)

mit Piperazin oder Piperazinderivaten der Formel (VII)

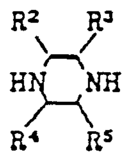

(VII)

hergestellt werden. Man arbeitet hierbei in einem Verdünnungsmittel, wie z.B. Dimethylsulfoxid, Hexamethylphosphorsäuretrsamid, Sulfolan, Wasser, einem Alkohol oder Pyridin, bei Temperaturen von 20° bis 200°C, vorzugsweise bei 80° bis 180°C. Bei der Durchführung des Verfahrens setzt man auf 1 Mol Carbonsäure (VI) 1 bis 15 Mol der Verbindung (VII), vorzungsweise 1 bis 6 Mol der Verbindung (VII) ein. Bei Verwendung äquivalenter Mengen der Carbonsäure (VI) und des Piperazinderivates (VII) führt man die Umsetzung in Gegenwart eines säurebindenden Mittles, beispielsweise Triethylamin, 1,4-Diaza-bicyclo-[2,2,2]octan oder 1,8-Diaza-bicyclo[5,4,0]undec-7-en durch.

Die als Zwischenprodukt verwendete 7-Chlor-1-cyclopropyl-6-fluor-1,4-dihydro-4-oxo-3-chinolincarbonsäure der Formel (VIa) (VI; X = F) kann gemäß folgendem Reaktionsschema hergestellt werden:

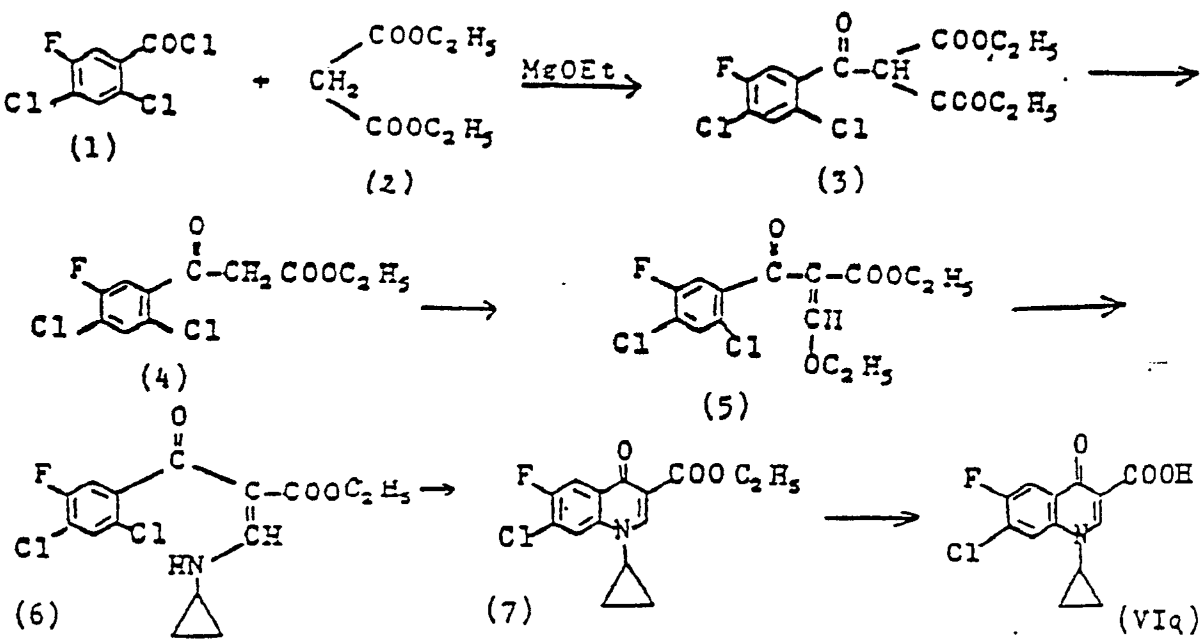


(1) (2) (3) (4) (5) (6) (7) (VIa)

Danach wird Malonsäurediethylester (2) mit 2,4-Dichlor-5-fluor-benzoylchlorid (1) in Gegenwart von Magnesium-alkoholat (Deutsche Patentanmeldung Nr. 3 142 856.8) zum Acylmalanester (3) acyliert (Organikum, 3. Auflage, *1964*, S. 438).

Durch partielle Verseifung und Decarboxylierung von (3) in wäßrigem Medium it katalytischen Mengen p-Toluol-sulfonsäure erhält man in guter Ausbeute den Aroylessigsäureethylester (4), der mit o-Ameisensäuretriethylester/Acetanhydrid in den 2-(2,4-Dichlor-5-fluor-benzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol, führt in leicht exothermer Reaktion zum gewunschten Zwischenprodukt (6).

Die Cyclisierungsreaktion (6) → (7) wird in einem Temperaturbereich von etwa 60° bis 280°C, bevorzugt 80° bis 180°C durchgeführt.

Als Verdünnungsmittel können Dioxan, Dimethylsulfoxid, N-Methyl-pyrrolidon, Sulfolan, Hexamethylphosphorsäuretriamid und bevorzugt, N,N-Dimethylformamid verwendet werden.

Als Säurebinder kommen für diese Reaktionsstufe Kalium-tert.-butanolat, Butyl-lithium, Lithiumphenyl, Phenylmagnesiumbromid, Natriummethylat, Natriumhydrid und besonders bevorzugt Kalium- oder Natriumcarbonat in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zur 7-Chlor-1-cyclopropyl-6-fluor-1,4-dihydro-4-oxo-3-chinolincarbonsäure VIa.

Das als Ausgangsmaterial für diesen Syntheseweg verwendete 2,4-Dichlor-5-fluor-benzoylchlorid (1) und die entsprechende Carbonsäure sowie das für die Herstellung von (1) benötigte 3-Fluor-4,6-dichlortoluol (10) werden gemäß nachstehendem Formelschema, ausgehend von 2,4-Dichlor-5-methyl-anilin (8), hergestellt:

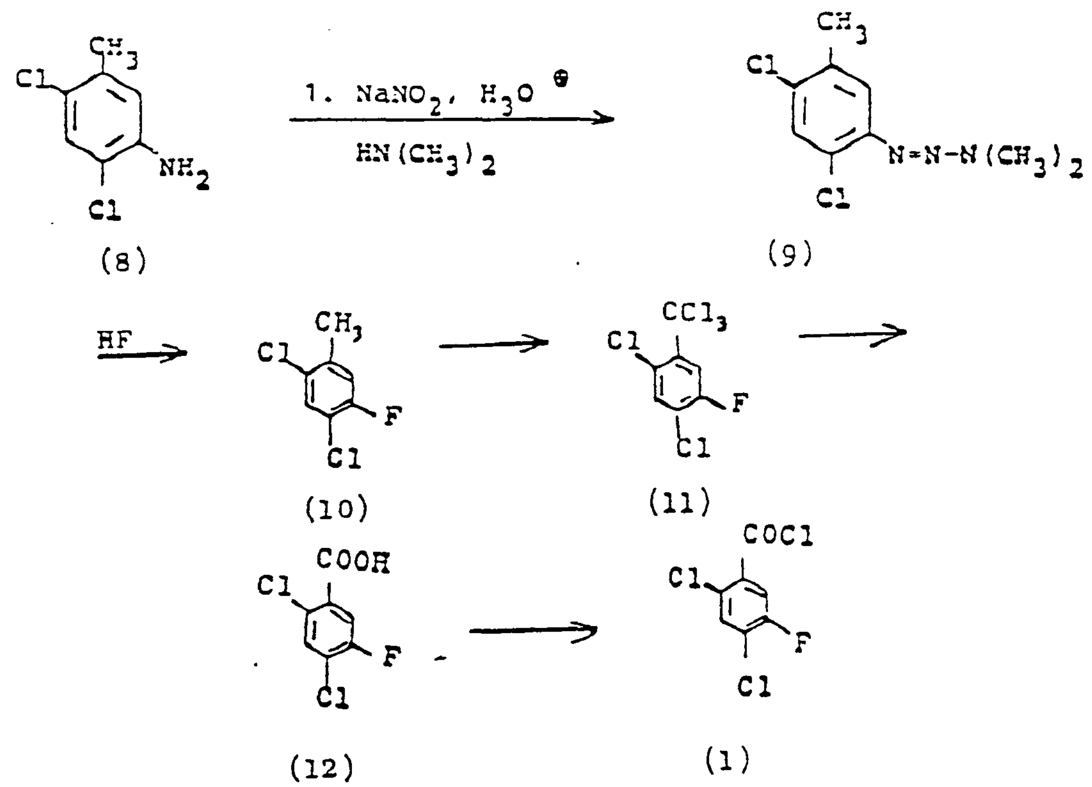

(8) (9) (10) (11) (12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazen (9) wird in überschüssiger wasserfreier Fluorwasserstoffsäure gelöst. Dabei spaltet das Triazen in 2,4-Dichlor-5-methyl-diazoniumfluorid und Dimethylamin. Ohne Zwischenisolierung wird diese Lösung thermisch bei 130—140° unter Stickstoff-Abspaltung in 3-Fluor-4,6-dichortoluol (10) gespalten. Ausbeute: 77% der Theorie.

Das 3-Fluor-4,6-dichlortoluol (10) wird in einem Temperaturbereich von 110 bis 160°C unter UV-Bestrahlung zu 2,4-Dichlor-5-fluor-1-trichlormethylbenzol (11) chloriert.

Die Verseifung von (11) mit 95%iger Schwefelsäure führt zu 2,4-Dichlor-5-fluor-benzoesäure (12), die mit Thionylchlorid in das Carbonsäurechlorid (1) (Siedepunkt 121°/20 mbar; $n_D^{20}$ 1,5722) übergeht.

Auf analoge Weise werden die folgenden als Zwischenprodukte verwendeten Chinoloncarbonsäuren hergestellt:

7-Chlor-1-cyclopropyl-1,4-dihydro-4-oxo-3-chinolincarbonsäure (VIb) (Schmelzpunkt 308°C) aus 2,4-Dichlorbenzoylchlorid (J. Chem. Soc. *83*, 1213 (1903));

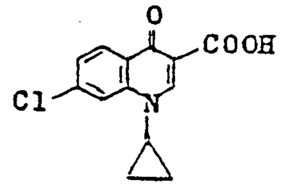

(VIb)

6,7-Dichlor-1-cyclopropyl-1,4-dihydro-4-oxo-3-chinolincarbonsäure (VIc) (Schmelzpunkt 265°C) aus 2,4,5-Trichlorbenzoylchlorid (Liebigs Ann. Chem. *152*, 238 (1869));

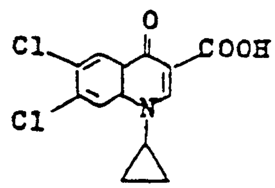

(VIc)

7-Chlor-1-cyclopropyl-1,4-dihydro-6-nitro-4-oxo-3-chinolincarbonsäure (VId) (Schmelzpunkt 265 bis 275°C Zers.) aus 2,4-Dichlor-5-nitro-benzopylchlorid (Liebigs Ann. Chem. *677*, 8 (1964)).

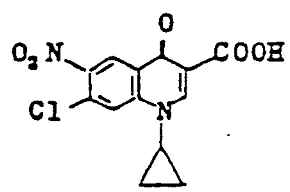

(VId)

Die als Ausgangsprodukte einzusetzenden Verbindung der Formel (III) sind bereits bekannt oder können nach an sich bekannten Verfahren erhalten werden, ebenso wie Verbindungen der Formel (IV).

Die erfindungsgemäß verwendbaren Verbindungen der Formel (V) können nach den oben beschriebenen Methoden A und B erhalten werden.

Die Umsetzung von (II) mit (III) (Methode A) wird vorzugsweise in einem Verdünnungsmittel, wie Dimethylsulfoxid, N,N-Dimethylformamid, Tetrahydrofuran, Sulfolan, Dioxan, Pyridin oder auch in Gemischen dieser Verdünnungsmittel, bei Temperaturen von 0°C bis 150°C, vorzungsweise 30°C bis 110°C, vorgenommen.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Als Saürebinder können alle üblichen anorganischen und organischen Säurebindungsmittel verwendet werden. Hierzu gehören vorzungsweise die Alkalihydroxide, Alkalicarbonate, Pyridin und tert.-Amine wie Triethylamin, 1,4-Diazabicyclo[2,2,2]octan. Die Umsetzung kann durch Zusatz von Kaliumiodid erleichtert werden.

Bei der Durchführung der Verfahrensvariante (A) setzt man auf 1 Mol der Verbindung (II) 1 bis 4 Mol, vorzungsweise 1 bis 1,5 Mol, der Verbindung (III) ein.

Die Umsetzung von (II) mit (IV) (Methode B) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Rekationstemperaturen können in einem größeren Bereich variiert werden. Im allgemeinen arbeitet man zwischen etwa 20°C und etwa 150°C, vorzugsweise zwischen 50°C und 100°C.

Die Umsetzung kann bei Normaldruck, aber auch bei erhöhtem Druck durchgeführt werden. Im allgemeinen arbeitet man bie Drucken zwischen etwa 1 und etwa 100 bar, vorzygsweise 1 und 10 bar.

Bei der Durchführung der Verfahrensvariante (B) setzt man auf 1 Mol der Verbindung (II) 1—5 Mol., vorzungsweise 1—2 Mol, der Verbindung (IV) ein.

Die Umsetzung der Verbindungen (V) zu den Dicarbonsäuren (Ib) (Methode C) erfolgt entweder in alkoholischer Natronlauge oder Kalilauge oder unter sauren Bedingungen in Mischungen von Schwefelsäure oder Chlorwasserstoff in Essigsäure und/oder Wasser. Die Hydrogenolyse von Benzylestern (V; R' = Benzyl) kann in Essigsäure in Gegenwart von Palladium-Katalysatoren erfolgen.

Man arbeitet im allgemeinen bei Temperaturen von 20°C bis 160°C, vorzugsweise bei 30 bis 140°C.

Die Umsetzung kann bie Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Die erfindungsgemäß verwendbaren Wirkstoffe weisen eine starke mikrobizide Wirkung auf und können zur Bekämpfung von unerwünschten Mikroorganismen praktisch eingesetzt werden. Die Wirkstoff sind für den Gebruach als Pflanzenschutzmittel geeignet.

Die erfindungsgemäß verwendbaren Verbindungen sind besonders gut wirksam gegen bakterielle Pflanzenkrankheiten.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die erfindungsgemäß zu verwendenden Verbindung zeigen auch eine fungizide Wirkung, z.B. gegen Pyricularia oryzae und Pellicularia sasakii im Reis.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bie normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als fest Trägerstoffe für Granulate kommen in Frage: z.B. gebrochene und fracktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen Fettsäureester, Polyoxyethylen-Fettaalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Caboxymethylcellulose, natürliche und synthetische pulverige, körnige oder latexförmige Polymere verwendet werden, wie Gummiarabicum, Polyvinylalkohol, Polyvinylacetate, sowie natürliche Phospholipe, wie Kephaline und Lecithine, und synthetische Phospholipide. Weitere Additive können minieralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Akariziden und Herbiziden, sowie in Mischungen mit Düngemitteln und Wachstumsregulatoren.

Die Wirkstoffe können als solche, in Form ihrer Formulierungen oder in daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auzubringen oder die Wirkstoffzubereitung oder den Wirkstoff selbst in den Boden zu injizieren. Es kann acuh das Saatgut der Pflanzen behandelt werdent.

Bei der Behandlung von Pflanzenteilen können dike Wirkstoffkonzentrationen in den Anwendungsformen in einem größeren Bereich variiert werden. Sie liegen im allgemeinen zwischen 1 und 0,0001 Gew.-%, vorzungsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzungsweise von 0.0001 bis 0,02%, am Wirkungsort erforderlich.

Herstellungsbeispiele

Ausgangsverbindungen:

Beispiel A

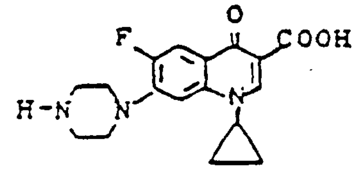

Ein Gemisch von 19,7 g 7-Chlor-1-cyclopropyl-6-fluor-1,4-dihydro-4-oxo-3-chinolincarbonsäure, 30,1 g wasserfreiem Piperazin und 100 ml Dimethylsulfoxid wird 2 Stunden auf 135 bis 140°C erhitzt. Das Lösungsmittel wird im Feinvakuum abdestilliert, der Rückstand in $H_2O$ suspendiert, abgesaugt und mit Wasser gewaschen. Zur weiteren Reinigung wird das feuchte Rohprodukt mit 100 ml Wassesr aufgekocht, bei Raumtemperatur abgesaugt, mit $H_2O$ gewaschen und im Vakuumtrockenschrank über $CaCl_2$ bei 100°C bis zur Gewichtskonstanz getrocknet. Man erhält 19,6 g 1-Cyclopropyl-6-fluor-1,4-dihydro-4-oxo-7-(1-piperazinyl)-3-chinolincarbonsäure vom Zersetzungspunkt 255 bis 257°C.

Die als Ausgangsmaterial verwendete 7-Chlor-1-cyclopropyl-6-fluor-1,4-dihydro-4-oxo-3-chinolincarbonsäure VIa wird wie folgt hergstellt:

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml absolutem Ethanol und 400 ml wasserfreiem Ether zu, wobei ein heftiger Rückfluß zu beobachten ist. Nach dem Abklingen der Reaktion wird noch 2 Stunden zum Sieden erhitzt, mit Trockeneis/Aceton auf −5°C bis −10°C gekühlt und bei dieser Temperatur eine Lösung von 227,5 g 2,4-Dichlor-5-fluor-benzoylchlorid (1) in 100 ml absolutem Ether langsam zugetropft. Man rührt 1 Stunde Bei 0°C bis −5°C, läßt über Nächt auf Raumtemperatur kommen und läßt unter Eiskühlung ein Gemisch von 400 ml Eiswasser und 25 ml konzentrierter Schwefelsäure zulaufen. Die Phasen werden getrennt und zweimal mit Ether nachextrahiert. Die vereinigten Etherlösungen werden mit gesättigter NaCl-Lösung gewaschen, mit $Na_2So_4$ getrocknet und das Lösungsmittel im Vakuum abgezogen. Man erhält 349,5 g 2,4-Dichlor-5-fluor-benzoyl-malonsäure-diethylester (3) als Rohprodukt.

Eine Emulsion von 34,9 g rohem 2,4-Dichlor-5-fluor-benzoylmalonsäure-diethylester (3) in 50 ml Wasser wird mit 0,15 g p-toluolsulfonsäure versetzt. Man erhitzt unter gutem Rühren 3.h zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstands im Vakuum liefert 21,8 g 2,4-Dichlor-5-fluorbenzoyl-essigsäureethylester (4) vom Siedepunkt 127 bis 142°C/0,09 mbar.

Ein Gemisch von 21,1 g 2,4-Dichlor-5-fluor-benzoyl-essigsäureethylester (4), 16,65 g o-Ameisensäureethylester und 18,55 g Acetanhydrid wird 2 Stunden auf 150°C erhitzt. Dann werden im Wasserstrahlvakuum und zuletzt im Feinvakuum bei einer Badtemperatur von 120°C die flüchtigen Bestandteile abdestilliert. Zurück bleiben 25,2 g roher 2-(2,4-Dichlor-5-fluor-benzoyl)-3-ethoxy-acrylsäureethylester (5). Er ist genügend rein für die weiteren Umnsetzungen.

Eine Lösung von 24,9 g 2-(2,4-Dichlor-5-fluor-benzoyl)-3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird nach 1 Stunde bei Raumtemperatur gerührt, das Lösungsmittel im Vakuum abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester (6) vom Schmelzpunkt 89 bis 90°C.

Eine Lösung von 31,9 g 2-(2,4-Dichlor-5-fluor-benzoyl)-3-cyclopropylamino-acylsäure-ethylester (6) in 100 ml wasserfreiem Dioxan wird unter Eiskühlung und Rühren portionsweise mit 3,44 g 80%igem Natriumhydrid versetzt. Dann wird 30 Minuten bei Raumtemperatur und 2 Stunden unter Rückfluß gerührt und das Dioxan im Vakuum abgezogen. Der Rückstand (40,3 g) wird in 150 ml Wasser suspendiert, mit 6,65 g Ätzkali versetzt und 1,5 h refluxiert. Man filtriert die warme Lösung und wäscht mit $H_2O$ nach. Dann wird mit halbkonzentrierter Salzsäure unter Eiskühlung auf pH = 1—2 angesäuert, der Niederschlag abgesaugt, mit Wasser gewaschen und im Vakuum bei 100°C getrocknet. Man erhält auf diese Weise 27,7 g 7-Chlor-1-cyclopropyl-6-fluor-1,4-dihydro-4-oxo-3-chinolincarbonsäure VIa vom Schmelzpunkt 234 bis 237°C.

Beispeil B

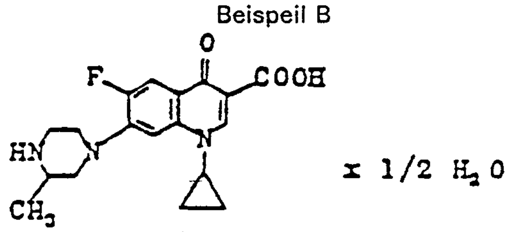

Ein Gemmisch aus 2,8 g (0.01 Mol) 7-Chlor-1-cyclopropyl-6-fluor-1,4-dihydro-4-oxo-3-chinolincarbonsäure und 5,1 g (0,051 Mol) 2-Methylpiperazin in 6 ml Dimethylsulfoxid wird 2 Stunden auf 140°C

erhitzt. Anschließend wird das Lösungsmittel im Hochvakuum abdestilliert, der Rückstand mit 6 ml heißem Wasser versetzt und 1 Stunde auf 95°C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90 bis 100°C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52% der Theorie) 1-Cyclopropyl-6-fluor-1,4-dihydro-4-oxo-7-(3-methyl-1-piperazinyl)-3-chinolincarbonsäure-semihydrat mit einem Zersetzungspunkt von 230 bis 232°C.

Beispiel C

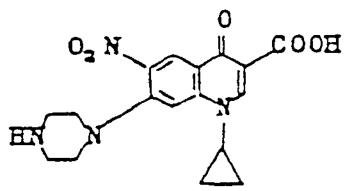

Eine Mischung von 9,3 g (0,03 Mol) 7-Chlor-1-cyclopropyl-1,4-dihydro-6-nitro-4-oxo-3-chinolincarbonsäure und 12,9 g (0,15 mol) Piperazin wird in 60 ml Dimethylsulfoxid 15 Minuten auf 120°C erwärmt. Aus der heißen Lösung fällt nach kurzer Zeit ein Niederschlag aus. Man engt im Hochvakuum ein, verrührt mit 30 ml Wasser und erhitzt noch 30 Minuten auf 95°C. Mit 2n-Salzsäure wird die Mimschung auf pH 8 eingestellt, der Niederschlag abgesaugt und mit Wasser und Methanol gewaschen. Man isoliert 5,8 g (54% der Theorie) 1-Cyclopropyl-1,4-dihydro-6-nitro-4-oxo-7-(1-piperazinyl)-3-chinolincarbonsäure mit einem Zersetzungspunkt von 296 bis 298°C.

Beispiel D

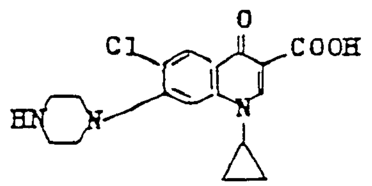

Man setzt analog Beispiel C 6,7-Dichlor-1-cyclopropyl-1,4-dihydro-4-oxo-3-chinoloncarbonsäure zu 1-Cyclopropyl-6-chlor-1,4-dihydro-4-oxo-7-(1-piperazinyl)-3-chinolincarbonsäure mit einem Zersetzungspunkt von 295 bis 298°C um.

Beispiel E

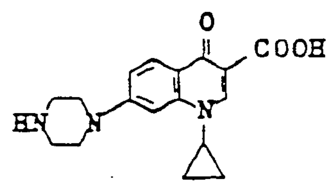

Man setzt analog Beispiel C 7-Chlor-1-cyclopropyl-1,4-dihydro-4-oxo-3-chinolincarbonsäure mit Piperazin zu 1-Cyclopropyl-1,4-dihydro-4-oxo-7-(1-piperazinyl)-3-chinolincarbonsäure mit einem Zersetzungspunkt von 298 bis 300°C um.

Endprodukte:

Beispiel 1

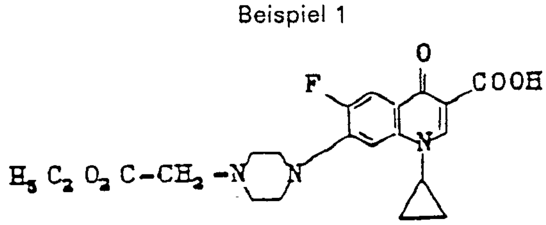

3,3 g (0.01 Mol) 1-Cyclopropyl-6-fluor-1,4-dihydro-4-oxo-7-(1-piperazinyl)-3-chinolincarbonsäure werden in 50 ml Dimethylformamid mit 2,5 g (0.015 Mol) Bromessigsäureethylester, 2,1 g (0,02 Mol) Triethylamin und 2,5 g Kaliumiodid 5 Stunden auf 90°C erhitzt. Die Reaktionsmischung wird in 30 ml Wasser eingegossen, der Niederschlag abgesaugt, mit Wasser gewaschen und aus Methanol umkristallisiert. man erhält 2,5 g 1-Cyclopropyl-6-fluor-7-[4-(ethoxycarbonylmethyl)-1-piperazinyl]-1,4-dihydro-4-oxo-3-chinolincarbonsäure mit einem Schmelzpunkt von 192 bis 194°C.

Analog Beispiel 1 werden folgende Verbindungen erhalten:

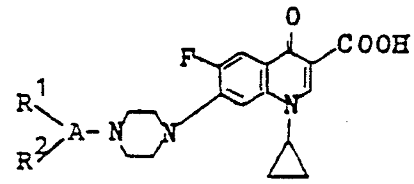

| Beispiel | $R^1$<br>A—<br>$R^2$ | Schmelzpunkt (°C) |
|---|---|---|
| 2 | $C_6H_5$—$CH_2O$—CO—CH—<br>⋮<br>$C_6H_5$ | 170 (Zers.) |
| 3 | $H_2N$—CO—$CH_2$— | 254 (Zers.) |
| 4 | NC—$CH_2$— | 166 (Zers.) |

Beispiel 5

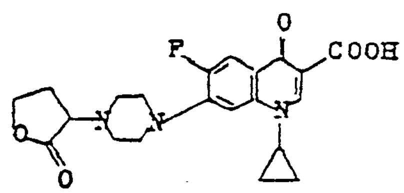

x HCl

Man arbeitet analog Beispiel 1 mit α-Brombutyrolacton als Alylierungsmittel, behandelt das Reaktionsprodukt mit verdünnter Salzsäure und erhält 1-Cyclopropyl-6-fluor-1,4-dihydro-4-oxo-7-[4-(2-oxo-tetrahydrofuryl-3-)-1-piperazinyl]-3-chinolincarbonsäure-hydrochlorid mit einem Zersetzungspunkt von 270°C. Massenspektrum: m/e 415 ($M^+$), 371, 342, 331, 301, 298, 289, 287, 275, 257, 245, 229, 36 (100%, HCl).

Beispiel 6

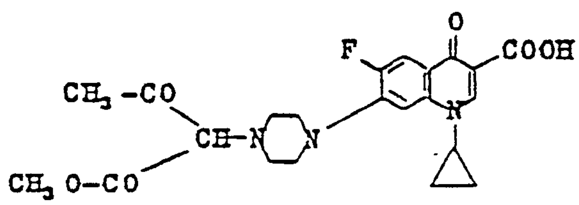

6,6 g (0,02 Mol) 1-Cyclopropyl-6-fluor-1,4-dihydro-4-oxo-7-(1-piperazinyl)-3-chinolincarbonsäure werden mit 4,5 g 2-Chloracetessigsäuremethylester und 4,2 g Triethylamin in 100 ml Dimethylformamid 3 Stunden auf 80°C erhitzt. Anschließend wird die Lösung im Vakuum eingeengt, mit 50 ml Wasser verrührt, das erhaltene Festprodukt mit Wasser und Methanol gewaschen und aus Glykolmonomethylether umkristallisiert. Man isoliert 3,9 g (44% der Theorie) 1-Cyclopropyl-6-fluor-1,4-dihydro-4-oxo-7-{4-[2-oxo-1-(methoxycarbonyl)-1-propyl]-1-piperazinyl}-3-chinolincarbonsäure mit einem Zersetzungspunkt von 224 bis 228°C.

Beispiel 7

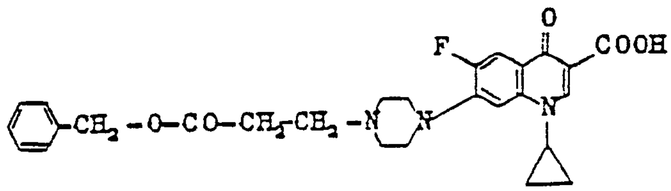

xHJ

3,3 g (0,01 Mol) 1-Cyclopropyl-6-fluor-1,4-dihydro-4-oxo-7-(1-piperazinyl)-3-chinolincarbonsäure

werden in 50 ml Dimethylformamid mit 5,8 g 3-Iodpropionsäurebenzylester und 2,1 g Triethylamin 2½ Stunden unter Rühren auf 70 bis 80°C erhitzt. Die Lösung wird im Vakuum eingeengt, mit 30 ml Wasser versetzt und auf pH 5 eingestellt. Der Niederschlag wird abgesaugt und mit Methanol ausgekocht, wobei 2,8 g 7-[4-(2-Benzyloxycarbonylethyl)-1-piperazinyl]-1-cyclopropyll-6-fluor-1,4-dihydro-4-oxo-3-chinolincarbonsäure-hydroiodid mit einem Zersetzungspunkt von 206 bis 210°C erhalten werden.

Der als Ausgangsstoff verwendete 3-Iodpropionsäurebenzylester wird auf folgendem Weg erhalten:

99 g 3-Chlorpropionsäurebenzylester werden mit 90 g Natriumiodid in 460 ml Aceton 1 Tag zum Rückfluß erhitzt. Das Reaktionsgemisch wird eingeengt, mit 200 ml Methylenchlorid versetzt und mit 3 × 100 ml Wasser gewaschen. Nach dem Trocknen mit Natriumsulfat wird eingeengt und der Rückstand im Hochvakuum derstilliert; Ausbeute: 91 g 3-Iodpropionsäurebenzylester mit einem Siedepunkt von 105 bis 108°C/13,3322 PA.

Beispiel 8

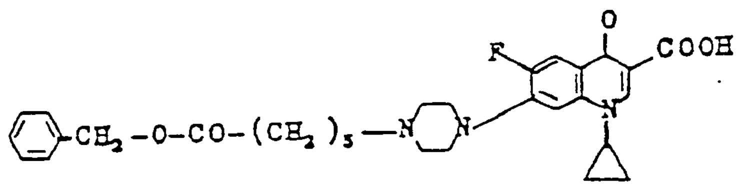

Man arbeitet analog Beispiel 7 mit 6-Iodhexansäurebenzylester und erhält 7-[4-(5-Benzyloxycarbonylpentyl)-1-piperazinyl]-1-cyclopropyl-6-fluor-1,4-dihydro-4-oxo-3-chinolincarbonsäure mit einem Schmelzpunkt von 176 bis 178°C.

Der als Ausgangsprodukt verwendete 6-Iodhexansäurebenzylester wird auf folgendem Weg erhalten:

46,5 g (0,3 Mol) 6-Chlorhexansäure und 35,6 g Benzylalkohol werden in 50 ml Toluol in Gegenwart von 1 g p-Toluolsulfonsäure am Wasserabscheider erhitzt. Nach beendeter Reaktion wäscht man mit 5% Natriumbicarbonatlösung und Wasser, trocknet mit Natriumsulft, engt ein und destilliert den Rückstand, wobei 61,5 g (85% der Theorie) 6-Chlorhexansäurebenzylester, Siedepunkt 163 bis 165°C/533,288 PA erhalten werden.

60 g (0,25 Mol) 6-Chlorhexansäurebenzylester werden mit 45 g Natriumiodid in 230 ml Aceton 5 Stunden unter Rückfluß erhitzt. Die Suspension wird engeengt, mit 300 ml Methylenchlorid versetzt und mit 2 × 200 ml Wasser gewaschen. Die organische Phase trocknet man mit Natriumsulfat, engt ein und destilliert den Rückstand in einer Kugelrohrdestillationsapparatur. Bei 220 bis 230°C (Ofentemperatur)/ 53,3288 PA gehen 63,8 g (77% der Theorie) 6-Iodhexansäurebenzylester über.

Beispeil 9

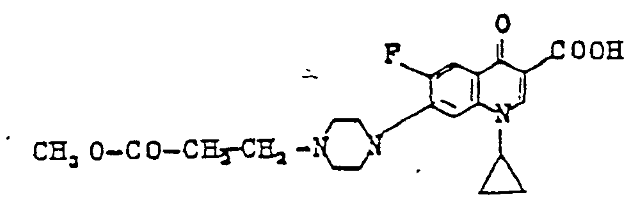

Eine Mischung von 3,31 g (0,01 Mol) 1-Cyclopropyl-6-fluor-1,4-dihydro-4-oxo-7-(1-piperazinyl)-3-chinolincarbonsäure und 5 g (0,058 Mol) Acrylsäuremethylester in 50 ml Ethanol wird 2 Stunden unter Rückfluß erhitzt. Die Lösung wird in 10 ml Wasser eingegossen, der Niederschlag abgesaugt, mit Methanol gewaschen und aus Glkyolmonomethylether umkristallisiert. Man erhält 2,9 g (70% der Theorie) 1-Cyclopropyl-6-fluor-1,4-dihydro-4-oxo-7-[4-(2-methoxycarbonylethyl)-1-piperazinyl]-3-chinolincabonsäure mit einem Zersetzungspunkt von 192 bis 194°C.

Analog Beispiel 9 werden folgende Verbindungen erhalten:

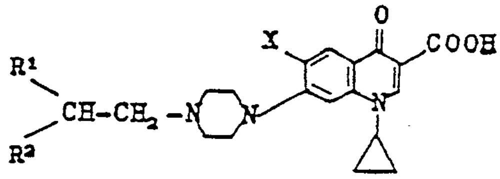

| Beispiel | $R^1$ | $R^2$ | X | Schmelzpunkt (°C) |
|---|---|---|---|---|
| 10 | $C_2H_5O—CO—$ | H | F | 142 (Zers.) |
| 11 | $C_4H_9O—CO—$ | H | F | 141 (Zers.) |
| 12 | $C_6H_5—CH_2O—CO—$ | H | F | 140 |
| 13 | $CH_3O—CO—$ | H | Cl | 183 |
| 14 | CN | H | F | 255 (Zers.) +) |
| 15 | CN | Cl | F | 202 (Zers.) ++) |
| 16 | $CH_3—SO_2—$ | H | F | 258 (Zers.) |

+) Die 7-[4-(2-Cyanoethyl)-1-piperazinyl)]-1-cyclopropyl-6-fluor-1,4-dihydro-4-oxo-3-chinolincarbonsäure liegt nach dem $^1$H-Kernresonanzspektrum im Gemisch mit ~15% 7-[4-(1-Cyanoethyl)-1-piperazinyl]-1-cyclopropyl-6-fluor-1,4-dihydro-4-oxo-3-chinolincarbonsäure vor.

++) Massenspektrum: m/e 382 ($M^+$—HCL), 338 (382—$CO_2$), 331, 289, 287, 245, 218, 154, 152, 44 ($CO_2$), 36 (100%, HCl).

## Beispiel 17

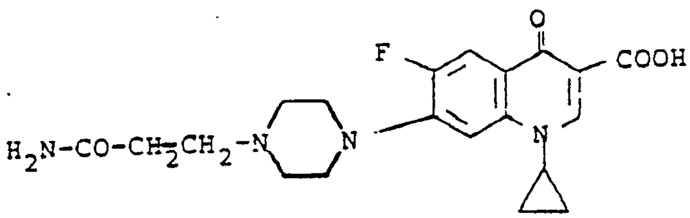

Eine Mischung von 3,31 g (0,01 Mol) 1-Cyclopropyl-6-fluor-1,4-dihydro-4-oxo-7-(1-piperazinyl)-3-chinolincarbonsäure und 4,2 g (0,058 Mol) Acrylamid in 50 ml Dimethylformamid wird 6 Stunden auf 140°C erhitzt. Die Suspension wird im Hochvakuum eingeengt, der Rückstand mit Wasser verrührt und aus Glykolmonomethylether umkristallisiert. Man erhält 2 g (50% der Theorie) 7-[4-(2-Carbamoylethyl)-1-piperazinyl]-1-cyclopropyl-6-fluor-1,4-dihydro-4-oxo-3-chinolincarbonsäure vom Zersetzungspunkt 283 bis 286°C.

## Beispiel 18

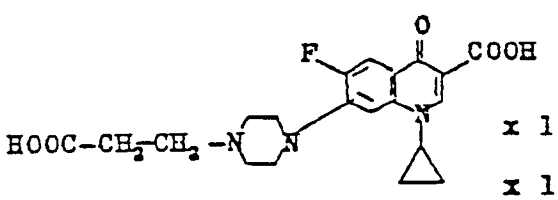

x 1/2 $H_2SO_4$

x 1/2 $H_2O$

2,9 g der Verbindung des Beispiels I9 werden in einer Mischung von 14 ml Essigsäure und 9,5 ml Wasser gelöst un mit 1,4 ml konsentrierter Schwefelsäure versetzt. Man erhitzt 1,5 Stunden auf 150 bis 160°C und gießt die Mischung in 90 ml Wasser ein. Der Niederschlag wird abgesaugt, mit Wasser und Methanol gewaschen und getrocknet. Man isoliert 2,3 g (72% der Theorie) 7-[4-(2-Carboxyethyl)-1-piperazinyl]-1-cyclopropyl-6-fluor-1,4-dihydro-4-oxo-3-chinolincarbonsäure-semisulfat-semihydrat vom Zersetzungspunkt 258 bis 261°C.

$C_{20}H_{22}FN_3O_5 \cdot \frac{1}{2}H_2SO_4 \cdot \frac{1}{2}H_2O$ (461,4)

| | | | | |
|---|---|---|---|---|
| berechnet: | C 52,06 | H 5,24 | N 9,11 | S 3,47 |
| gefunden: | C 51,7 | H 5,3 | N 9,1 | S 3,9 |

Analog Beispiel 12 werden folgende Verbindungen erhalten:

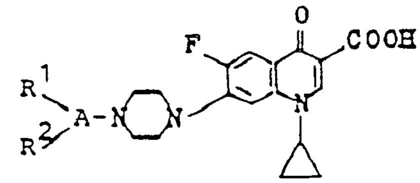

| Beispiel | $R^1$, $R^2$ >A— | Schmelzpunkt (°C) |
|---|---|---|
| 19 | $HOOC-CH_2-$ × $2\frac{1}{2}H_2O$ | 276 (Zers.)[1] |
| 20 | $HOOC-(CH_2)_5-$ × $\frac{1}{2}H_2SO_4$ × $\frac{1}{2}H_2O$ | 254 (Zers.) |
| 21 | $HOOC-CH(C_6H_5)-$ × $H_2O$ | 214 (Zers.)[2] |

[1] Das Reaktionsprodukt (als Sulfat) wird in verdünnter Natronlauge gelöst und mit verdünnter Salzsäure bei pH 5 als Betain ausgefällt.
[2] Das auf Wasser gegebene Reaktionsgemisch wird mit verdünnter Natronlauge auf pH 4 engestellt und das Betain isoliert.

Beispeil 22

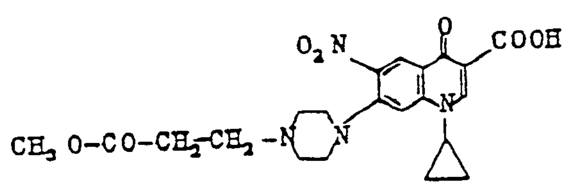

537 mg (0,0015 Mol) 1-Cyclopropyl-1,4-dihydro-6-nitro-4-oxo-7-(1-piperazinyl)-3-chinolincarbonsäure werden in einer Mischung aus 7,5 ml Glykolmonomethylether and 3 ml Dimethylsulfoxid mit 2 g Acrylsäuremethylester 8 Stunden unter Rückfluß erhitzt. Die Lösung wird mit 10 ml Wasser versetzt, der Niederschlag abgesaugt, mit Methanol gewaschen und getrocknet. Man erhält 0,5 g 1-Cyclopropyl-1,4-dihydro-7-[4-(2-methoxycarbonylethyl)-1-piperazinyl]-6-nitro-4-oxo-3-chinolincarbonsäure mit einem Zersetzungspunkt von 208 bis 211°C.

Beispiel 23

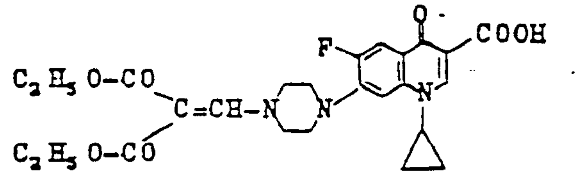

In einer Mischung aus 0,4 g Natriumhydroxid in 5 ml Wasser und 25 ml Dioxan werden 3,3 g 1-Cyclopropyl-6-fluor-1,4-dihydro-4-oxo-7-(1-piperazinyl)-3-chinolincarbonsäure mit 2,8 g Ethoxymethylenmalonsäurediethylester 5 Stunden bei Raumtemperatur gerührt. Man läßt über Nacht stehen, filtriert von wenig Ungelöstem ab und engt das Filtrat ein. Der Rückstand wird in ca. 30 ml Wasser aufgenommen, mit verdünnter Salzsäure auf pH 4 eingestellt und der ausgefallene Niederschlag sofort abgesaugt und mit Wasser gewaschen. Man erhält ein schmieriges Produkt, das sich durch Verrühren mit Isopropanol verfestigt.

Ausbeute: 2,4 g (48% der Theorie) 1-Cyclopropyl-7-[4-(2,2-Diethoxycarbonyl-vinylen)-1-piperazinyl]-6-fluor-1,4-dihydro-4-oxo-3-chinolincarbonsäure mit einem Zersetzungspunkt von 184 bis 188°C.

Beispiel 24

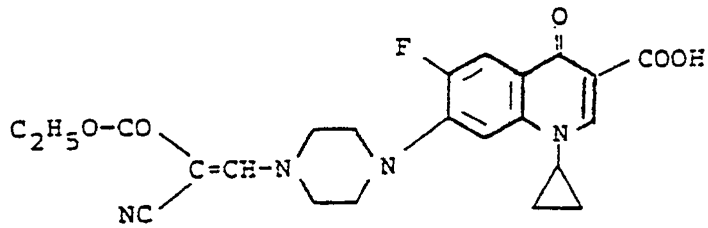

Man arbeitet analog Beispiel 23, jedoch mit 2,2 g Ethoxymethylencyuanessigsäureethylester und erhält 2,35 g 1-Cyclopropyl-7-[4-(2-cyano-2-ethoxycarbonylvinylen)-1-piperazinyl]-6-fluor-1,4-dihydro-4-oxo-3-chinolincarbonsäure mit einem Zersetzungspunkt von 245 bis 255°C.

Beispiel 25

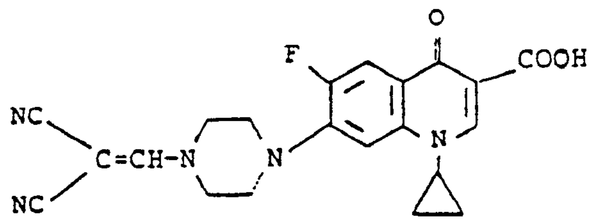

Man arbeitet analog Beispiel 23, jedoch mit 1,6 g Ethoxymethylenmalonsäuredinitril und erhält 4 g 1-Cyclopropyl-7-[4-(2,2-dicyano-vinylen)-1-piperazinyl]-6-fluor-1,4-dihydro-4-oxo-3-chinolincarbonsäure als schwerlösliches Produkt, das mit Ethanol gewaschen wird; Zersetzungspunkt 275 bis 283°C.

Massenspektrum: m/e = 363 ($M^+$ —$CO_2$), 362 ($M^+$ —COOH), 315, 287, 245, 44 (100%, $CO_2$).

Beispiel 26

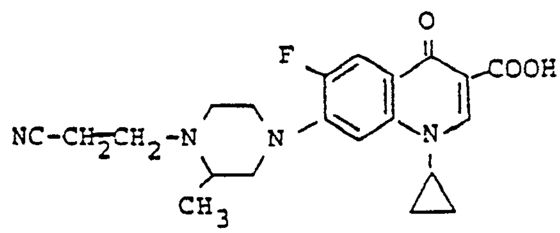

Man setzt analog Beispiel 9 3,45 g 1-Cyclopropyl-6-fluor-1,4-dihydro-4-oxo-7-(3-methyl-1-piperazinyl)-3-chinolincarbonsäure (Beispiel B) mit 4,5 g Acylnitril um und erhält 3 g 7-[4-(2-Cyanoethyl)-3-methyl-1-piperazinyl]-1-cyclopropyl-6-fluor-1,4-dihdyro-4-oxo-3-chinolincarbonsäure mit einem Schmelzpunkt von 203—106°C.

$C_{21}H_{23}FN_4O_3$ (398,4)

| | | | |
|---|---|---|---|
| berechnet: | C, 63,3 | H 5,8 | N 14,1 |
| gefunden: | C 63,0 | H 5,9 | N 13,8 |

Verwendungsbeispiele

In den nachfolgenden Beispielen wird die nachstehend angegebene Verbindung also Vergleichssubstanz eingesetzt:

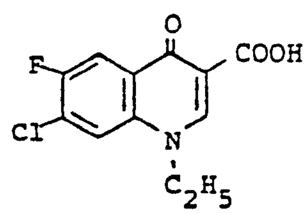

Beispiel I

Xanthomonas oryzae-Tesdt/Bakteriose/Reis/systemisch

Lösungsmittel: 48,5 Gewichtsteile Dimethylformamid

Emulgator: 1,5 Gewichtsteile Alkylarylpolyglykolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wäßrigen Suspension von Xanthomonas oryzae durch Stichverletzung inokuliert. Danach verbleiben die Pflanzen 14 Tage bis zur Auswertung in einem Gewächshaus bei 24 bis 26°C und 70 bis 80% rel. Luftfeuchtigkeit.

Eine deutliche Überlegenheit in der Wirksamkeit gegenüber dem Stand der Technik zeigen bei diesem Test z.B. die Verbindungen gemäß folgender Herstellungsbeispiele:

TABELLE A

Xanthomonas oryzae-Test / Bakteriose / Reis / systemisch

| Wirkstoffe | Aufwandmenge in mg Wirkstoff pro 100 $cm^2$ | Krankheitsbefall in % der unbehandelten Kontrolle systemisch |
|---|---|---|
| 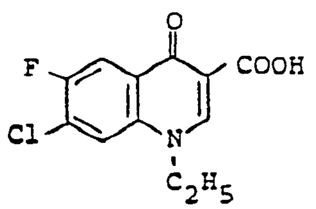 (bekannt) | 10 | 60 |
| 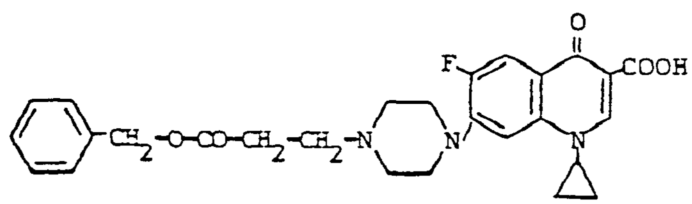 · HJ | 10 | 40 |

Beispiel II

Agarplattentest

Verwendeter Nährboden

15 Gewichtsteile Agar-Agar
10 Gewichtsteile Saccharose
8 Gewichtsteile Caseinhydrolysat
4 Gewichtsteile Hefeextrakt
2 Gewichtsteile Dikaliumhydrogenphosphate
0,3 Gewichtsteile Magnesiumphosphate

werden in 1000 ml destilliertem Wasser gelöst und 15 Minuten bei 121°C autiklaviert.

Lösungsmittel: 10 Gewichtsteile Dimethylformamid

Mengenverhältnis von Lösungsmittel zu Nährboden: 0,2:99,8

Zur Herstellung einer zweckmäßigen Wirkstoffzubereitung vermischt man 1 Gewichtsteil Wirkstoff mit der angegebenen Menge Lösungsmittel.

Das Konzentrat wird im gennanten Mengenverhältnis mit dem flüssigen Nährboden gründlich vermischt und in Pertrischalen gegossen.

Ist der Nährboden erkaltet und fest, werden die Platten mit folgenden Mikroorganismen beimpft und bei ca. 28°C inkubiert:

Die Auswertung erfolgt nach 2 Tagen, wobei die Wachstumshemmung als Maß für die Wirkung der Präparate herangezogen wird.

Eine deutliche Überlegenheit gegenüber dem Stand der Technkik zwigen in diesem Test die Verbindungen gemäß folgender Herstellungsbeispiele:

TABELLE B

| Agarplatten-Test<br>Wirkstoffe | Wirkstoff-konzentration ppm | Wachstum in Wertzahlen (1 = kein Wachstum, 9 = Kontrolle) Mikroorganismen<br>Agrobacterium tumefaciens | Corynebacterium michiganense |
|---|---|---|---|
| 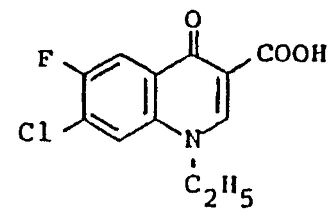 (bekannt) | 10 | 4 | 4 |
| 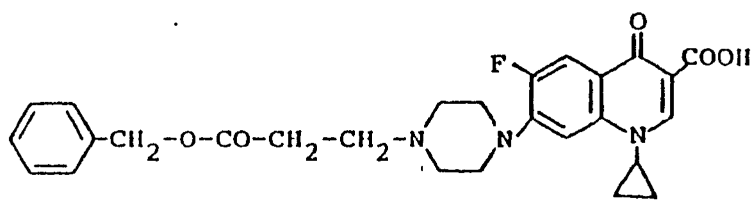 ·HJ | 10 | 3 | 2 |
| 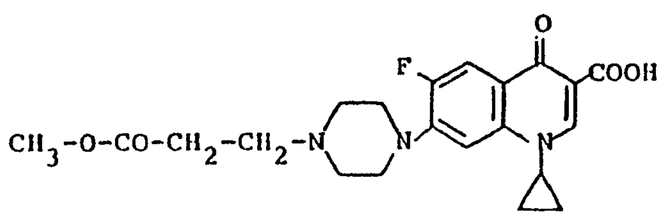 | 10 | 3 | 2 |

TABELLE B (Fortsetzung)

| Agarplatten-Test<br>Wirkstoffe | Wirkstoff-konzentration ppm | Wachstum in Wertzahlen (1 = kein Wachstum, 9 = Kontrolle) Mikroorganismen<br>Agrobacterium tumefaciens | <br>Corynebacterium michiganense |
|---|---|---|---|
| 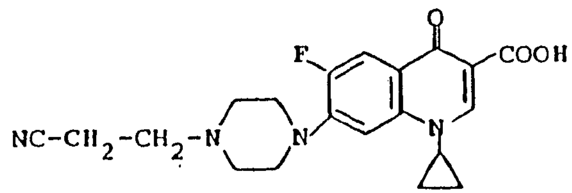 | 10 | 3 | 3 |
| 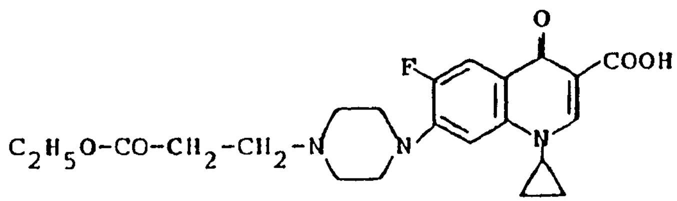 | 10 | 3 | 3 |
| 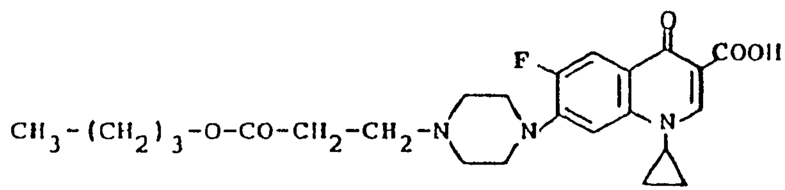 | 10 | 3 | 3 |
| 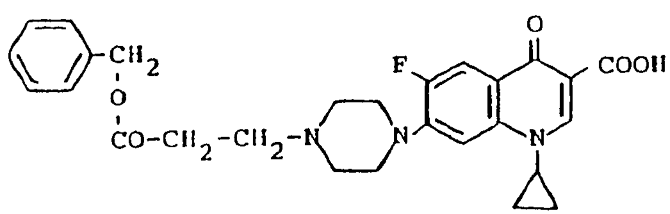 | 10 | 3 | 3 |

**Patentansprüche**

1. Verwendung von Chinoloncarbonsäuren der Formel (I)

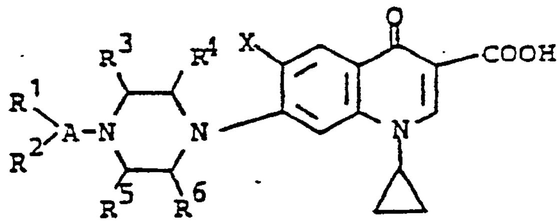

(I)

in der

A für geradkettiges oder verzweigtes Alkylen mit 1 bis 6 Kohlenstoffatomen oder einen Rest >C=CH—,

$R^1$ für Alkoxycarbonyl mit 1 bis 6 Kohlenstoffatomen im Alkylteil, Benzyloxycarbonyl, Carboxy, gegebenenfalls substituiertes Carbamoyl, Cyano, Dialkoxyphosphonyl oder Alkylsulfonyl mit 1 bis 4 Kohlenstoffatomen im Alkylteil und

$R^2$ für Wasserstoff, Alkoxycarbonyl mit 1 bis 6 Kohlenstoffatomen im Alkylteil, Benzyloxycarbonyl, gegebenenfalls substituiertes Carbamoyl, Cyano, Chlor, Acetyl, Alkyl mit 1 bis 3 Kohlenstoffatomen oder Phenyl steht, wobei $R^1$ und $R^2$ auch gemeinsam mit dem Kohlenstoffatom, das sie substituieren, einen 2-Oxo-tetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen oder Nitro steht,

und deren pflanzenverträglichen Säureadditions-, Alkali- und Erdalkalisalze, Zink-, Mangan- und Kupfersalze und Hydrate zur Bekämpfung von Bakterien im Pflanzenschutz.

2. Verwendung der Verbindung der Formel (I) gemäß Anspruch 1, in der

A für geradkettiges oder verzweigtes Akylen mit 1 bis 5 Kohlenstoffatomen oder die Gruppe >C=CH— steht,

$R^1$ für Alkoxycarbonyl mit 1 bis 5 Kohlenstoffatomen im Alkylteil, Benzyloxycarbonyl, Carboxy, gegebenefalls durch 1 oder 2 Methyl- oder Ethylreste substituiertes Carbamoyl, Cyano, Methyl- oder Ethylsulfonyl steht,

$R^2$ für Wasserstoff, Alkoxycarbonyl mit 1 bis 5 Kohlenstoffatomen im Alkylteil, Benzyloxycarbonyl, Carbamoyl, Cyano, Chlor, Acetyl, Alkyl mit 1 oder 2 Kohlenstoffatomen oder Phenyl steht, außerdem $R^1$ und $R^2$ auch gemeinsam mit dem Kohlenstoffatom, das sie substituieren, einen 2-Oxo-tetrahydrofurylring bilden können,

$R^3$, $R^4$, $R^5$ und $R^6$ für Wasserstoff, Methyl oder Ethyl stehen und

X für Wasserstoff, Fluor, Chlor oder Nitro

stehen, zur Bekämpfung von Bakterien im Pflanzenschutz.

3. Verwendung der Verbindung der Formel (I) in Anspruch 1, in der

A für geradkettiges Alkylen mit 1 bis 5 Kohlenstoffatomen oder die Gruppe >C=CH— steht,

$R^1$ für Alkoxycarbonyl mit 1 bis 4 Kohlenstoffatomen im Alkylteil, Benzyloxycarbonyl, Carboxy, Carbamoyl, Cyano oder Methylsulfonyl steht,

$R^2$ für Wasserstoff, Alkoxycarbonyl mit 1 bis 3 Kohlenstoffatomen im Alkylteil, Cyano, Chlor, Acetyl oder Phenyl steht, außerdem $R^1$ und $R^2$ auch gemeinsam mit dem Kohlenstoffatom, das sie substituieren, einen 2-Oxo-tetrahydro-3-furylring bilden können,

$R^3$ für Wasserstoff, Methyl oder Ethyl steht,

$R^4$ für Wasserstoff steht,

$R^5$ für Wasserstoff oder Methyl,

$R^6$ für Wasserstoff und

X für Wasserstoff, Fluor, Chlor oder Nitro

stehen, zur Bekämpfung von Bakterien im Pflanzenschutz.

4. Zink-, Mangan- oder Kupfersalze der Chinoloncarbonsäuren der Formel (I')

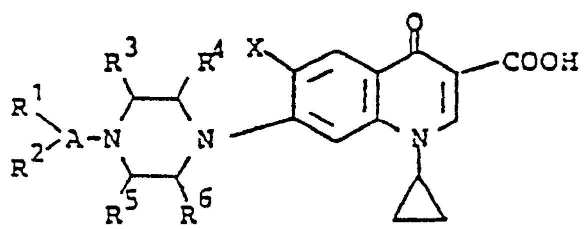

(I')

in der

A für geradkettiges oder verzweites Alkylen mit 1 bis 6 Kohlenstoffatomen oder einen Rest >C=CH—,

$R^1$ für Alkoxycarbonyl mit 1 bis 6 Kohlenstoffatomen im Alkylteil, Benzyloxycarbonyl, Carboxy, gegebenenfalls substituiertes Carbamoyl, Cyano, Dialkoxyphosphonyl oder Alkylsulfonyl mit 1 bis 4 Kohlenstoffatomen im Alkylteil und

$R^2$ für Wasserstoff, Alkoxycarbonyl mit 1 bis 6 Kohlenstoffatomen im Alkylteil, Benzyloxycarbonyl, gegebenenfalls substituiertes Carbamoyl, Cyano, Chlor, Acetyl, Alkyl mit 1 bis 3 Kohlenstoffatomen oder Phenyl stehen, wobei $R^1$ und $R^2$ auch gemeinsam mit dem Kohlenstoffatom, an das sie gebunden sind einen 2-Oxo-tetrahydrofurylring bilden können und

$R^3$, $R^4$, $R^5$ und $R^6$ gleich oder verschieden sein können und für Wasserstoff, Methyl, Ethyl, n- oder i-Propyl stehen, und

X für Wasserstoff, Halogen oder Nitro steht.

5. Zink-, Mangan- und Kupfersalze der Verbindungen der Formel (I') gemäß Anspruch 4, worin

A für geradkettiges oder verzweigtes Akylen mit 1 bis 5 Kohlenstoffatomen oder die Gruppe >C=CH— steht,

$R^1$ für Alkoxycarbonyl mit 1 bis 5 Kohlenstoffatomen im Alkylteil, Benzyloxycarbonyl, Carboxy, gegebenefalls durch 1 oder 2 Methyl- oder Ethylreste substituiertes Carbamoyl, Cyano, Methyl- oder Ethylsulfonyl steht,

$R^2$ für Wasserstoff, Alkoxycarbonyl mit 1 bis 5 Kohlenstoffatomen im Alkylteil, Benzyloxycarbonyl, Carbamoyl, Cyano, Chlor, Acetyl, Alkyl mit 1 oder 2 Kohlenstoffatomen oder Phenyl steht, wobei $R^1$ und $R^2$ auch gemeinsam mit dem Kohlenstoffatom, das sie substituieren, einen 2-Oxo-tetrahydrofurylring bilden können,

$R^3$, $R^4$, $R^5$ und $R^6$ für Wasserstoff, Methyl oder Ethyl stehen und

X für Wasserstoff, Fluor, Chlor oder Nitro steht.

6. Zink-, Mangan- oder Kupfersalze der Verbindungen der Formel (I'), nach Anspruch 4, in der

A für geradkettiges Alkylen mit 1 bis 5 Kohlenstoffatomen oder die Gruppe >C=CH— steht,

$R^1$ für Alkoxycarbonyl mit 1 bis 4 Kohlenstoffatomen im Alkylteil, Benzyloxycarbonyl, Carboxy, Carbamoyl, Cyano oder Methylsulfonyl steht,

$R^2$ für Wasserstoff, Alkoxycarbonyl mit 1 bis 3 Kohlenstoffatomen im Alkylteil, Cyano, Chlor, Acetyl oder Phenyl steht, wobei $R^1$ und $R^2$ auch gemeinsam mit dem Kohlenstoffatom, das sie substituieren, einen 2-Oxo-tetrahydro-3-furylring bilden können,

$R^3$ für Wasserstoff, Methyl oder Ethyl steht,

$R^4$ für Wasserstoff steht,

$R^5$ für Wasserstoff oder Methyl steht,

$R^6$ für Wasserstoff und

X für Wasserstoff, Fluor, Chlor oder Nitro stehen.

## Claims

1. Use of quinolonecarboxylic acids of the formula (I)

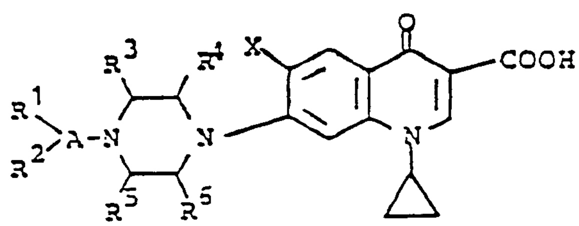

(I)

in which

A represents straight-chain or branched alkylene with 1 to 6 carbon atoms or a >C=CH— radical,

$R^1$ represents alkoxycarbonyl with 1 to 6 carbon atoms in the alkyl part, benzyloxycarbonyl, carboxyl, optionally substituted carbamoyl, cyano or dialkylphosphonyl or alkylsulphonyl with 1 to 4 carbon atoms in the alkyl part,

$R^2$ represents hydrogen, alkoxycarbonyl with 1 to 6 carbon atoms in the alkyl part, benzyloxycarbonyl, optionally substituted carbamoyl, cyano, chlorine, acetyl, alkyl with 1 to 3 carbon atoms or phenyl, or

$R^1$ and $R^2$, together with the carbon atom which they substitute, can also form a 2-oxo-tetrahydrofuryl ring, and

$R^3$, $R^4$, $R^5$ and $R^6$ can be identical or different and represent hydrogen, methyl, ethyl or n- or i-propyl and

X represents hydrogen, halogen or nitro,

and their plant-tolerated acid addition salts, alkali metal salts and alkaline earth metal salts, zinc, manganese and copper salts and hydrates for combating bacteria in plant protection.

2. Use of the compounds of formula (I) according to Claim 1, in which

A represents straight-chain or branched alkylene with 1 to 5 carbon atoms or the >C=CH— group,

$R^1$ represents alkoxycarbonyl with 1 to 5 carbon atoms in the alkyl part, benzyloxycarbonyl, carboxyl, carbamoyl which is optionally substituted by 1 or 2 methyl or ethyl radicals, cyano, methylsulphonyl or ethylsulphonyl,

$R^2$ represents hydrogen, alkoxycarbonyl with 1 to 5 carbon atoms in the alkyl part, benzyloxycarbonyl, carbamoyl, cyano, chlorine, acetyl, alkyl with 1 or 2 carbon atoms or phenyl, or

$R^1$ and $R^2$, together with the carbon atom which they substitute, can also form a 2-oxo-tetrahydrofuryl ring,

$R^3$, $R^4$, $R^5$ and $R^6$ represent hydrogen, methyl or ethyl and

X represents hydrogen, fluorine or nitro, for combating bacteria in plant protection.

3. Use of the compounds of the formula (I) in Clailm 1, in which

A represents straight-chain alkylene with 1 to 5 carbon atoms or the >C=CH— group,

$R^1$ represents alkoxycarbonyl with 1 to 4 carbon atoms in the alkyl part, benzyloxycarbonyl, carboxyl, carbamoyl, cyano or methylsulphonyl,

$R^2$ represents hydrogen, alkoxycarbonyl with 1 to 3 carbon atoms in the alkyl part, cyano, chlorine, acetyl or phenyl, or

$R^1$ and $R^2$, together with the carbon atom which they substitute, can also form a 2-oxo-tetrahydro-3-furyl ring,

$R^3$ represents hydrogen, methyl or ethyl,

$R^4$ represents hydrogen,

$R^5$ represents hydrogen or methyl,

$R^6$ represents hydrogen and

X represents hydrogen, fluorine, chlorine or nitro,

for combating bacteria in plant protection.

4. Zinc, manganese or copper salts of the quinolonecarboxylic acids of the formula (I')

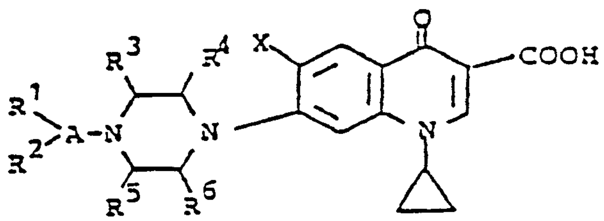

(I')

in which

A represents straight-chain or branched alkylene with 1 to 6 carbon atoms or a >C=CH— radical,

$R^1$ represents alkoxycarbonyl with 1 to 6 carbon atoms in the alkyl part, benzyloxycarbonyl, carboxyl, optionally substituted carbamoyl, cyano or dialkylphosphonyl or alkylsulphonyl with 1 to 4 carbon atoms in the alkyl part,

$R^2$ represents hydrogen, alkoxycarbonyl with 1 to 6 carbon atoms in the alkyl part, benzyloxycarbonyl, optionally substituted carbamoyl, cyano, chlorine, acetyl, alkyl with 1 to 3 carbon atoms or phenyl, or

$R^1$ and $R^2$, together with the carbon atom to which they are bonded, can also form a 2-oxo-tetrahydrofuryl ring,

$R^3$, $R^4$, $R^5$ and $R^6$ can be identical or different and represent hydrogen, methyl, ethyl or n- or i-propyl and

X represents hydrogen, halogen or nitro.

5. Zinc, manganese and copper salts of the compounds of the formula (I') according to Claim 4, wherein

A represents straight-chain or branched alkylene with 1 to 5 carbon atoms or the >C=CH— group,

$R^1$ represents alkoxycarbonyl with 1 to 5 carbon atoms in the alkyl part, benzyloxycarbonyl, carboxyl, carbamoyl which is optionally substituted by 1 or 2 methyl or ethyl radicals, cyano, methylsulphonyl or ethylsulphonyl,

$R^2$ represents hydrogen, alkoxycarbonyl with 1 to 5 carbon atoms in the alkyl part, benzyloxycarbonyl, carbamoyl, cyano, chlorine, acetyl, alkyl with 1 or 2 carbon atoms or phenyl, or

$R^1$ and $R^2$, together with the carbon atom which they substitute, can also form a 2-oxo-tetrahydrofuryl ring,

$R^3$, $R^4$, $R^5$ and $R^6$ represent hydrogen, methyl or ethyl and

X represents hydrogen, fluorine, chlorine or nitro.

6. Zinc, manganese or copper salts of the compounds of the formula (I'), acocrding to Claim 4, in which

A represents straight-chain alkylene with 1 to 5 carbon atoms or the >C=CH— group,

$R^1$ represents alkoxycarbonyl with 1 to 4 carbon atoms in the alkyl part, benzyloxycarbonyl, carboxyl, carbamoyl, cyano or methylsulphonyl,

$R^2$ represents hydrogen, alkoxycarbonyl with 1 to 3 carbon atoms in the alkyl part, cyano, chlorine, acetyl or phenyl, or

$R^1$ and $R^2$, together with the carbon atom which they substitute, can also form a 2-oxo-tetrahydro-3-furyl ring,

$R^3$ represents hydrogen, methyl or ethyl,

$R^4$ represents hydrogen,

$R^5$ represents hydrogen or methyl,

$R^6$ represents hydrogen and

X represents hydrogen, fluorine, chlorine or nitro.

**Revendications**

1. Utilisation d'acides quinolone-carboxyliques de formule (I):

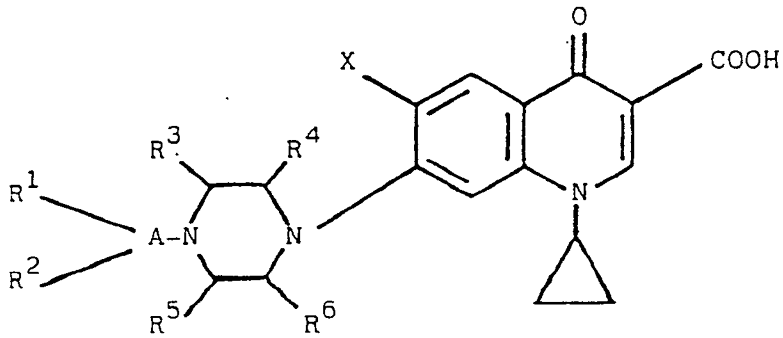

dans laquelle

A représente un groupe alkylène à chaîne droite ou ramifée contenant 1 à 6 atomes de carbone ou un radical >C=CH—,

$R^1$ représente un groupe alcoxycarbonyle contenant 1 à 6 atomes de carbone dans la fraction alkyle, un groupe benzyloxycarbonyle, un groupe carboxy, un group carbamoyle èventuellement substitué, un groupe cyano, un groupe dialcoxyphosphonyle ou un groupe alkylsulfonyle contenant 1 à 4 atomes de carbone dans la fraction alkyle,

$R^2$ représente un atome d'hydrogène, un groupe alcoxycarbonyle contenant 1 à 6 atomes de carbone dans la fraction alkyle, un groupe benzyloxycarbonyle, un groupe carbamoyle èventuellement substitué, un groupe cyano, un atome de chlore, un groupe acétyle, un groupe alkyle contenant 1 à 3 atomes de carbone ou un groupe phényle, $R^1$ et $R^2$, ensemble avec l'atome de carbone qu'ils substituent, pouvant également former un noyau 2-oxo-tétrahydrofuryle, tandis que

$R^3$, $R^4$, $R^5$ et $R^6$ peuvent être identiques ou différents et représentent chacun un atome d'hydrogène, un groupe méthyle, un groupe éthyle, un groupe n-propyl ou un groupe i-propyle, et

X représente un atome d'hydrogène, un atome d'halogène ou un groupe nitro,

ainsi que de leurs hydrates, leurs sels de zinc, de manganèse et de cuivre, leurs sels de métaux alcalins et de métaux alcalino-terreux et de leurs sels d'addition d'acides compatibles avec les plantes en vue de combattre les bactéries dans le domaine de la protection des plantes.

2. Utilisation des composés de formule (I) selon la revendication 1, formule dans laquelle

A représente un groupe alkylène à chaîne droite ou ramifée contenant 1 à 5 atomes de carbone ou le groupe >C=CH—,

$R^1$ représente un groupe alcoxycarbonyle contenant 1 à 5 atomes de carbone dans la fraction alkyle, un groupe benzoyloxycarbonyle, un groupe carboxy, un groupe carbamoyle éventuellement substitué par un ou deux radicaux méthyle ou éthyle, un groupe cyano, un groupe méthyl- ou éthyle-sulfonyle,

$R^2$ représente un atome d'hydrogène, un groupe alcoxycarbonyle contenant 1 à 5 atomes de carbone dans la fraction alkyle, un groupe benzyloxycarbonyle, un groupe carbamoyle, un groupe cyano, un atome de chlore, un groupe acétyle, un groupe alkyle contenant 1 ou 2 atomes de carbone ou un groupe phényle tandis que, en outre, $R^1$ et R.305, ensemble avec l'atome de carbone qu'ils substituent, peuvent former un noyau 2-oxo-tétrahydrofuryle,

$R^3$, $R^4$, $R^5$ et $R^6$ représentent chacun un atome d'hydrogène, un groupe méthyle ou un groupe éthyle, et

X représente un atome d'hydrogène, un atome de fluor, ou un groupe nitro,

en vue de combattre les bactéries dans le domaine de la protection des plantes.

3. Utilisation des composés de formule (I) selon la revendication 1, formule dans laquelle

A représente un groupe alkylène à chaîne droite contenant 1 à 5 atomes de carbone ou le groupe >C=CH—,

$R^1$ représente un groupe alcoxycarbonyle contenant 1 à 4 atomes de carbone dans la fraction alkyle, un groupe benzyloxycarbonyle, un groupe carboxy, un groupe carbamoyle, un groupe cyano ou un groupe méthylsulfonyle,

$R^2$ représente un atome d'hydrogène, un groupe alcoxycarbonyle contenant 1 à 3 atomes de carbone dans la fraction alkyle, un groupe cyano, un atome de chlore, un groupe acétyle ou un groupe phényle tandis que, en outre, $R^1$ et $R^2$, ensemble avec l'atome de carbone qu'ils substituent, peuvent également former un noyau 2-oxo-tétrahydro-3-furyle,

$R^3$ représente un atome d'hydrogène, un groupe méthyle, ou un groupe éthyle,

$R^4$ représente un atome d'hydrogène,

$R^5$ représente un atome d'hydrogène ou un groupe méthyle,

$R^5$ représente un atome d'hydrogène et

X représente un atome d'hydrogène, un atome de fluor, un atome de chlore ou un groupe nitro,

en vue de combattre les bactéries dans le domaine de la protection des plantes.

4. Sels de zinc, de manganése ou de cuivre des acides quinolone-carboxyliques de formule (I'):

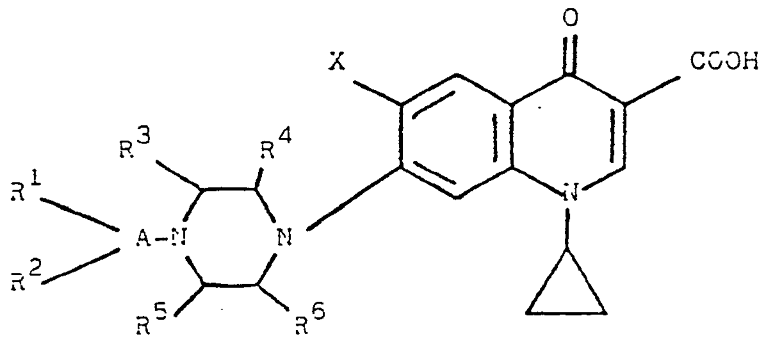

(I')

dans laquelle

A représente un groupe alkylène à chaîne droite ou ramifiée contenant 1 à 6 atomes de carbone ou un radical >C=CH—,

$R^1$ représente un groupe alcoxycarbonyle contenant 1 à 6 atomes de carbone dans la fraction alkyle, un groupe benzyloxycarbonyle, un groupe carboxy, un group carbamoyle èventuellement substitué, un groupe cyano, un groupe dialcoxyphosphonyle ou un groupe alkylsulfonyle contenant 1 à 4 atomes de carbone dans la fraction alkyle, et

$R^2$ représente un atome d'hydrogène, un groupe alcoxycarbonyle contenant 1 à 6 atomes de carbone dans la fraction alkyle, un groupe benzyloxycarbonyle, un groupe carbamoyle èventuellement substitué, un groupe cyano, un atome de chlore, un groupe acétyle, un groupe alkyle contenant 1 à 3 atomes de carbone ou un groupe phényle, $R^1$ et $R^2$ pouvant également former, ensemble avec l'atome de carbone auquel ils sont reliés, un noyau 2-oxo-tétrahydrofuryle, et

$R^3$, $R^4$, $R^5$ et $R^6$ peuvent être identiques ou différents et représentent chacun un atome d'hydrogène, un groupe méthyle, un groupe éthyle, un groupe n-propyl ou un groupe i-propyle, et

X représente un atome d'hydrogène, un atome d'halogène ou un groupe nitro.

5. Sels de zinc, de manganèse et de cuivre des composés de formule (I') selon la revendication 4, formule dans laquelle

A représente un groupe alkylène à chaîne droite ou ramifée contenant 1 à 5 atomes de carbone ou le groupe >C=CH—,

$R^1$ représente un groupe alcoxycarbonyle contenant 1 à 5 atomes de carbone dans la fraction alkyle, un groupe benzoyloxycarbonyle, un groupe carboxy, un groupe carbamoyle éventuellement substitué par un ou deux radicaux méthyle ou éthyle, un groupe cyano, un groupe méthyl- ou éthyle-sulfonyle,

$R^2$ représente un atome d'hydrogène, un groupe alcoxycarbonyle contenant 1 à 5 atomes de carbone dans la fraction alkyle, un groupe benzyloxycarbonyle, un groupe carbamoyle, un groupe cyano, un atome de chlore, un groupe acétyle, un groupe alkyle contenant 1 ou 2 atomes de carbone ou encore un groupe phényle, $R^1$ et $R^2$, ensemble avec l'atome de carbone qu'ils substituent, pouvant également former un noyau 2-oxo-tétrahydrofuryle,

$R^3$, $R^4$, $R^5$ et $R^6$ représentent chacun un atome d'hydrogène, un groupe méthyle ou un groupe éthyle, et

X représente un atome d'hydrogène, un atome de fluor, un atome de chlore ou un groupe nitro.

6. Sels de zinc, de manganèse ou de cuivre des composés de formule (I') selon la revendication 4, formule dans laquelle

A représente un groupe alkylène à chaîne droite contenant 1 à 5 atomes de carbone ou le groupe >C=CH—,

$R^1$ représente un groupe alcoxycarbonyle contenant 1 à 4 atomes de carbone dans la fraction alkyle, un groupe benzoyloxycarbonyle, un groupe carboxy, un groupe carbamoyle, un groupe cyano ou un groupe méthylsulfonyle,

$R^2$ représente un atome d'hydrogène, un groupe alcoxycarbonyle contenant 1 à 3 atomes de carbone dans la fraction alkyle, un groupe cyano, un atome de chlore, un groupe acétyle ou un groupe phényle, $R^1$ et $R^2$, ensemble avec l'atome de carbone qu'ils substituent, pouvant également former un noyau 2-oxo-tétrahydro-3-furyle,

$R^3$ représente un atome d'hydrogène, un groupe méthyle, ou un groupe éthyle,

$R^4$ représente un atome d'hydrogène,

$R^5$ représente un atome d'hydrogène ou un groupe méthyle,

$R^5$ représente un atome d'hydrogène et

X représente un atome d'hydrogène, un atome de fluor, un atome de chlore ou un groupe nitro.